# EUROPÄISCHE PATENTSCHRIFT

(11) **EP 0 480 270 B1**
(45) Veröffentlichungstag und Bekanntmachung des Hinweises auf die Patenterteilung: **04.05.1994**
(21) Anmeldenummer: 91116615.5
(22) Anmeldetag: 28.09.1991
(51) Int. Cl.: C07C 323/62, C07C 319/20, G03G 9/097, C25D 13/10

(54) **Arylsulfidverbindungen sowie Verfahren zu ihrer Herstellung**
Arylsulfides and process for their fabrication
Arylsulfures ainsi que procédé pour leur fabrication

(30) Priorität: 06.10.1990 DE 4031704; 21.03.1991 DE 4109261
(43) Veröffentlichungstag der Anmeldung: 15.04.1992
(73) Patentinhaber: CASSELLA Aktiengesellschaft, D-60386 Frankfurt (DE)
(72) Erfinder: Nagl, Gert, Dr., W-6369 Niederdorfelden (DE); Macholdt, Hans-Tobias, Dr., W-6100 Darmstadt-Eberstadt (DE)
(74) Vertreter: Muley, Ralf, Dr.

(56) Entgegenhaltungen:
- EP-A- 201 340
- EP-A- 0 085 181
- EP-A- 0 181 526
- EP-A- 0 299 329
- CH-A- 131 285
- CH-A- 131 289
- US-A- 2 614 120
- US-A- 4 480 021
- Beilstein's Handbuch der Organischen Chemie,vierte Auflage, Band 10 (1927), Seiten 148-149

## Beschreibung

Gegenstand der vorliegenden Erfindung sind neue Arylsulfidverbindungen sowie Verfahren zu ihrer Herstellung.

Die vorliegende Erfindung betrifft Verbindungen der allgemeinem Formel I 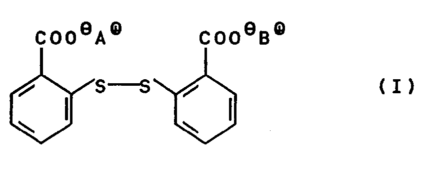
worin
A^{⊕} und B^{⊕} unabhängig voneinander mit der Einschränkung, daß sie nicht beide gleichzeitig Protonen bedeuten, ein Proton oder ein Ion der allgemeinen Formel II 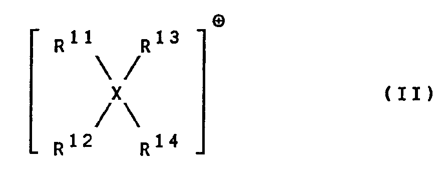
worin X für Stickstoff oder Phosphor steht; und R¹¹, R¹², R¹³ und R¹⁴ unabhängig voneinander mit der Einschränkung, daß sie nicht alle gleichzeitig für Wasserstoff stehen, für Wasserstoff; (C₁-C₃₀)-Alkyl; (C₅-C₁₂)-Cycloalkyl; (C₆-C₁₂)-Aryl oder für (C₆-C₁₂)-Aryl-(C₁-C₆)-alkyl stehen.

Alkylgruppen können geradkettig oder verzweigt sein. Für sowie R¹¹ bis R¹⁴ stehende Alkylgruppen haben bevorzugt 1 bis 22 Kohlenstoffatome. Besonders bevorzugt sind Methyl, Ethyl, Propyl, Butyl, Pentyl, Hexyl, Heptyl, Octyl, Nonyl, Decyl, Dodecyl sowie Hexadecyl.

(C₅-C₁₂)-Cycloalkyl kann einkernig oder mehrkernig sein und bedeutet bevorzugt Cyclopentyl und Cyclohexyl.

(C₆-C₁₂)-Aryl kann einkernig oder mehrkernig sein und bedeutet bevorzugt Phenyl, 1-Naphthyl, 2-Naphthyl und Biphenyl.

(C₆-C₁₂)-Aryl-(C₁-C₆)alkyl bedeutet bevorzugt Benzyl.

In der allgemeinen Formel II bedeutet X besonders bevorzugt Stickstoff. Besonders bevorzugte Verbindungen der allgemeinen Formel I sind solche mit A^{⊕} = B^{⊕} und solche mit A^{⊕} = H^{⊕}.

Ganz besonders bevorzugte Verbindungen der allgemeinen Formel I sind solche der allgemeinen Formel Ib 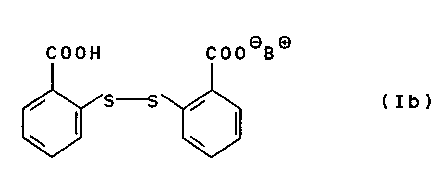
worin B^{⊕}
Tetramethylammonium
Tetraethylammonium
Tetrapropylammonium
Tetrabutylammonium
Tetrapentylammonium
Tetrahexylammonium
Tetraheptylammonium
Tetraoctylammonium
Tetranonylammonium
Tetrakis-(decyl)-ammonium
Tetradodecylammonium
Tributylmethylammonium
Benzyltrimethylammonium
Ethylhexadecyldimethylammonium
Benzyldimethylhexadecylammonium
Benzyltriethylammonium
Hexadecyltrimethylammonium
Tetrapropylphosphonium
Tetrabutylphosphonium
Tetrapentylphosphonium
Benzyltriphenylphosphonium oder
Hexadecyltributylphosphonium
bedeutet,
und solche der allgemeinen Formel Ic 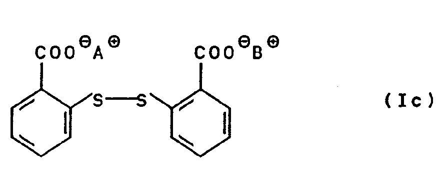
worin A^{⊕} und B^{⊕} identisch sind und
Tetramethylammonium
Tetraethylammonium
Tetrapropylammonium
Tetrabutylammonium
Tetrapentylammonium
Tetrahexylammonium
Tetraheptylammonium
Tetraoctylammonium
Tetranonylammonium
Tetrakis-(decyl)-ammonium
Tetradodecylammonium
Tributylmethylammonium
Benzyltrimethylammonium
Ethylhexadecyldimethylammonium
Benzyldimethylhexadecylammonium
Benzyltriethylammonium
Hexadecyltrimethylammonium
Tetrapropylphosphonium
Tetrabutylphosphonium
Tetrapentylphosphonium
Benzyltriphenylphosphonium oder
Hexadecyltributylphosphonium
bedeuten.

Erfindungsgemäße Verbindungen der allgemeinen Formel I sind beispielweise:
2,2'-Dithiodibenzoesäure-mono-(tetramethylammonium)salz
2,2'-Dithiodibenzoesäure-di-(tetramethylammonium)salz
2,2'-Dithiodibenzoesäure-mono-(tetraethylammonium)salz
2,2'-Dithiodibenzoesäure-di-(tetraethylammonium)salz
2,2'-Dithiodibenzoesäure-mono-(tetrapropylammonium)salz
2,2'-Dithiodibenzoesäure-di-(tetrapropylammonium)salz
2,2'-Dithiodibenzoesäure-mono-(tetrabutylammonium)salz
2,2'-Dithiodibenzoesäure-di-(tetrabutylammonium)salz
2,2'-Dithiodibenzoesäure-mono-(tetrapentylammonium)salz
2,2'-Dithiodibenzoesäure-di-(tetrapentylammonium)salz
2,2'-Dithiodibenzoesäure-mono-(tetrahexylammonium)salz
2,2'-Dithiodibenzoesäure-di-(tetrahexylammonium)salz
2,2'-Dithiodibenzoesäure-mono-(tetraheptylammonium)salz
2,2'-Dithiodibenzoesäure-di-(tetraheptylammonium)salz
2,2'-Dithiodibenzoesäure-mono-(tetraoctylammonium)salz
2,2'-Dithiodibenzoesäure-di-(tetraoctylammonium)salz
2,2'-Dithiodibenzoesäure-mono-(tetrakis-(decyl)-ammonium) salz
2,2'-Dithiodibenzoesäure-di-(tetrakis-(decyl)-ammonium)salz
2,2'-Dithiodibenzoesäure-mono-(tetradodecylammonium)salz
2,2'-Dithiodibenzoesäure-di-(tetradodecylammonium)salz
2,2'-Dithiodibenzoesäure-mono-(tributylmethylammonium)salz
2,2'-Dithiodibenzoesäure-di-(tributylmethylammonium)salz
2,2'-Dithiodibenzoesäure-mono-(benzyltrimethylammonium)-s alz
2,2'-Dithiodibenzoesäure-di-(benzyltrimethylammonium)salz
2,2'-Dithiodibenzoesäure-mono-(ethylhexadecyldimethylammonium)salz
2,2'-Dithiodibenzoesäure-di-(ethylhexadecyldimethylammonium)salz
2,2'-Dithiodibenzoesäure-mono-(benzyldimethylhexadecylammonium)salz
2,2'-Dithiodibenzoesäure-di-(benzyldimethylhexadecylammonium)salz
2,2'-Dithiodibenzoesäure-mono-(benzyltriethylammonium) salz
2,2'-Dithiodibenzoesäure-di-(benzyltriethylammonium)salz
2,2'-Dithiodibenzoesäure-mono-(hexadecyltrimethylammonium)salz
2,2'-Dithiodibenzoesäure-di-(hexadecyltrimethylammonium)salz
2,2'-Dithiodibenzoesäure-mono-(benzyltriphenylphosphonium)salz
2,2'-Dithiodibenzoesäure-di-(benzyltriphenylphosphonium)salz
2,2'-Dithiodibenzoesäure-mono-(tetrapropylphosphonium)salz
2,2'-Dithiodibenzoesäure-di-(tetrapropylphosphonium)salz
2,2'-Dithiodibenzoesäure-mono-(tetrabutylphosphonium)salz
2,2'-Dithiodibenzoesäure-di-(tetrabutylphosphonium)salz
2,2'-Dithiodibenzoesäure-mono-(tetrapentylphosphonium)salz
2,2'-Dithiodibenzoesäure-di-(tetrapentylphosphonium)salz
2,2'-Dithiodibenzoesäure-mono-(hexadecyltributylphosphonium)salz
2,2'-Dithiodibenzoesäure-di-(hexadecyltributylphosphonium)salz.

Die erfindungsgemäßen Verbindungen der allgemeinen Formel I können hergestellt werden durch Umsetzung der Verbindungen der allgemeinen Formel V 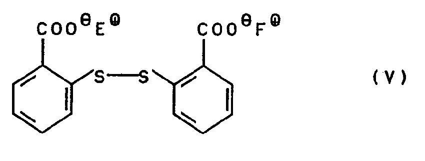
worin R¹ bis R⁸ und m und n die oben angegebene Bedeutung haben und E^{⊕} und F^{⊕} unabhängig voneinander für ein Proton, ein Alkaliion oder das Ammoniumion stehen, mit einer Verbindung der Formel A^{⊕}Yₐ^{⊖} oder B^{⊕}Y_{b}^{⊖} oder einem Gemisch beider Verbindungen,
worin Yₐ^{⊖} und Y_{b}^{⊖} unabhängig voneinander ein Anion oder ein Anionäquivalent bedeuten und A^{⊕} und B^{⊕} die oben angegebene Bedeutung haben, wobei das Proton ausgeschlossen ist.

Ein für E^{⊕} bzw. F^{⊕} stehendes Alkaliion ist bevorzugt das Natrium- oder das Kaliumion.
Anionen Yₐ^{⊖} und Y_{b}^{⊖} sind bevorzugt Hydroxid, Carbonat, Hydrogencarbonat, Chlorid, Bromid, Iodid, Tetrafluoroborat, Tosylat, Sulfat oder Hydrogensulfat.

In einer bevorzugten Ausführungsform des erfindungsgemäßen Verfahrens wird mit Hydroxiden AOH bzw. BOH gearbeitet, wobei ein Lösungsmittel gewählt wird, in dem sowohl Hydroxid als auch Reaktionsprodukt löslich sind. Lösungsmittel dieser Art sind organische Lösungsmittel, wobei Alkohole wie beispielsweise Methanol, Ethanol, 1-Propanol und 2-Propanol sowie deren Gemische mit Wasser besonders geeignet sind. E^{⊕} und F^{⊕} stehen dabei für ein Proton. Die Ausgangsverbindungen werden dabei in stöchiometrischen Mengen eingesetzt und die Produkte, die in reiner Form in der theoretischen Ausbeute anfallen, können direkt aus dem Reaktionsgemisch durch Einengen und anschließendes Trocknen erhalten werden.

Die Reaktionstemperatur ist dabei nicht kritisch. Zweckmäßigerweise arbeitet man in dem Temperaturbereich zwischen Raumtemperatur und dem Siedepunkt des Reaktionsgemisches.

In einer weiteren bevorzugten Ausführungsform wird das erfindungsgemäße Verfahren nach dem Prinzip einer Fällungsreaktion ausgeführt. Als Reaktionsmedium ist im Prinzip jedes sich an der Reaktion nicht beteiligende Lösungsmittel oder Lösungsmittelgemisch geeignet, in dem ein Dialkalisalz oder das Diammoniumsalz der Verbindung der allgemeinen Formel V löslich, das erfindungsgemäße Produkt der allgemeinen Formel I aber unlöslich ist. In der Regel ist als Reaktionsmedium Wasser besonders gut geeignet.

Vorteilhafterweise geht man dabei so vor, daß man die Verbindung der allgemeinen Formel V, worin E^{⊕} und F^{⊕} für ein Proton stehen, mit der berechneten Menge Alkali-oder Ammoniumhydroxid in Wasser zum Dialkali- bzw. Diammoniumsalz löst und mit einer Verbindung der Formel A^{⊕}Yₐ^{⊖} oder B^{⊕}Y_{b}^{⊖} oder einem Gemisch beider Verbindungen, bevorzugt in gelöster Form, versetzt. Erfindungsgemäße Produkte der allgemeinen Formel I, die in Wasser unlöslich sind, fallen dabei aus und können durch Abfiltrieren isoliert werden.

Die Temperatur ist auch bei diesem Verfahren nicht kritisch. Vorteilhafterweise liegt sie zwischen 5 und 95°C.

Für den Fall, daß ein erfindungsgemäßes Disalz der allgemeinen Formel I im Reaktionsmedium löslich, das entsprechende Monosalz aber unlöslich ist, kann letzteres in einfacher Weise durch Zugabe von Säure zu einer Lösung des Dialkali- oder des Diammoniumsalzes der allgemeinen Formel V und eines neutralen Salzes A^{⊕}Yₐ^{⊖} bzw. B^{⊕}Y_{b}^{⊖} ausgefällt und durch Abfiltrieren isoliert werden.

Wenn jedoch Yₐ^{⊖} bzw. Y_{b}^{⊖} für ein einbasigsaures Anion, beispielsweise Hydrogensulfat steht, fällt das unlösliche erfindungsgemäße Monosalz bereits bei der Zugabe von A^{⊕}Yₐ^{⊖} bzw. B^{⊕}Y_{b}^{⊖} zur Lösung des Dialkali- oder des Diammoniumsalzes der allgemeinen Formel V aus. In diesem Fall setzt man vorteilhafterweise höchstens die stöchiometrische Menge an A^{⊕}Yₐ^{⊖} bzw. B^{⊕}Y_{b}^{⊖} ein, um einen Überschuß an saurem Anion zu vermeiden. Im Falle neutraler Anionen setzt man zur Erzielung einer guten Ausbeute zweckmäßigerweise die stöchiometrische Menge oder einen kleinen Überschuß an A^{⊕}Yₐ^{⊖} bzw. B^{⊕}Y_{b}^{⊖} ein.

Die zur Ausfällung verwendetete Säure muß genügend stark sein. Geeignet sind beispielsweise Schwefelsäure, Salzsäure und Ameisensäure.

Zweckmäßigerweise verwendet man zur Ausfällung von Monosalzen der allgemeinen Formel I höchstens die stöchiometrische Menge an Säure. Zu wenig Säure verschlechtert die Ausbeute, zuviel Säure bewirkt eine Verunreinigung des Produkts mit entsprechender Dibenzoesäure.

Im Prinzip können die unlöslichen Monosalze der allgemeinen Formel I auch dadurch erhalten werden, daß eine Lösung der Verbindung A^{⊕}Yₐ^{⊖} bzw. B^{⊕}Y_{b}^{⊖} worin Yₐ^{#SDF#} bzw. Y_{b}^{⊖} für ein neutrales Anion steht, zu einer Lösung eines Monoalkali- oder Monoammoniumsalzes der allgemeinen Formel V gegeben wird. Das gewünschte Produkt fällt dabei zwar auch aus, in der Praxis hat diese Vorgehensweise aber den schwerwiegenden Nachteil, daß zur vollständigen Auflösung eines Monoalkalisalzes oder eines Monoammoniumsalzes zum Zwecke der glatten Umsetzung wesentlich größere Verdünnungen erforderlich sind.

Ein ganz besonders bevorzugtes Verfahren ist ein Verfahren zur Herstellung von in Wasser schwerlöslichen Monosalzen der allgemeinen Formel I, dadurch gekennzeichnet, daß sie aus einer wäßrigen Lösung, die eine Verbindung der allgemeinen Formel Va 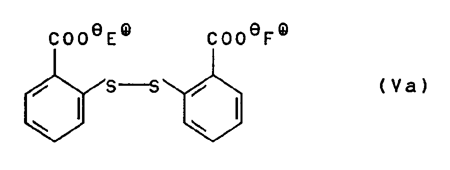
worin
E^{⊕} und F^{⊕} unabhängig voneinander für ein Alkaliion oder das Ammoniumion stehen und eine Verbindung A^{⊕}Yₐ^{⊖} oder B^{⊕}Y_{b}^{⊖} worin A^{⊕} und B^{⊕} wie in Anspruch 1 angegeben definiert sind und Yₐ^{⊖} und Y_{b}^{⊖} ein Anion oder ein Anionäquivalent bedeuten, in äquimolaren Mengen enthält, durch Kohlendioxid gefällt wird.

Dieses Verfahren ist besonders vorteilhaft zur Herstellung von Salzen der 2,2'-Dithiodibenzoesäure mit Ionen der allgemeinen Formel II, worin X Stickstoff oder Phosphor und R¹¹ bis R¹⁴ n-Propyl oder n-Butyl bedeuten.

Die Verbindung der allgemeinen Formel I wird bevorzugt durch Einleiten von gasförmigem Kohlendioxid gefällt. Dabei wird bevorzugt Kohlendioxid bis zu einem pH-Wert von 6,0 bis 9,0, besonders bevorzugt 7,0 bis 8,0, eingeleitet.

Die Temperatur ist nicht kritisch und liegt bevorzugt zwischen 5 und 95°C.

Das gefällte Produkt kann in einfacher und an sich bekannter Weise isoliert und getrocknet werden.

Die Verbindungen der Formeln A^{⊕}Yₐ^{⊖} und B^{⊕}Y_{b}^{⊖} sind im Handel erhältlich und/oder können nach dem Fachmann bekannten Methoden hergestellt werden.

Die Verbindungen der allgemeinen Formel V, worin E^{⊕} und F^{⊕} für ein Proton stehen, sind literaturbekannt und beispielsweise in folgender Literaturstelle beschrieben:
ORGANIC SYNTHESIS Collective Volume 2 (1943), 580
D.R.P 601642
DE-OS 3 201 904

Die erfindungsgemäßen Verbindungen der allgemeinen Formel I können außer in reiner Form auch in Form von Gemischen und Mischkristallen untereinander, sowie in Form von Gemischen und Mischkristallen mit bis zu 90 Gew.% der Verbindungen der allgemeinen Formel V, worin E^{⊕} und F^{⊕} für ein Proton stehen, vorliegen.

Die vorliegende Erfindung betrifft deshalb auch Gemische sowie Mischkristalle aus
a) 10 bis 100 Gew.% einer oder mehreren Verbindungen der allgemeinen Formel I und
b) 0 bis 90 Gew.% 2,2'-Dithiodibenzoesäure.

Bevorzugte Gemische sowie Mischkristalle sind solche aus zwei Verbindungen der allgemeinen Formel I, wobei Gemische sowie Mischkristalle aus Disalz und Monosalz besonders bevorzugt sind.

Bevorzugt sind auch Gemische und Mischkristalle einer Verbindung der allgemeinen Formel I mit A^{⊕} = H^{⊕} mit weniger als 50 Gew.%, besonders bevorzugt mit weniger als 20 Gew.% der entsprechenden Verbindung der allgemeinen Formel V, worin E^{⊕} und F^{⊕} für ein Proton stehen.

Die erfindungsgemäßen Gemische können hergestellt werden durch einfaches Mischen der entsprechenden Mengen der Einzelbestandteile.

Erfindungsgemäße Gemische sowie Mischkristalle können aber auch analog den oben für Einzelverbindungen angegebenen Verfahren erhalten werden, wobei entsprechende Gemische von Ausgangsverbindungen eingesetzt werden bzw. ein Überschuß an Säure verwendet wird.

Die erfindungsgemäßen Verbindungen sind hervorragend geeignet für den Einsatz als farblose Ladungssteuermittel in Tonern und Entwicklern für elektrophotographische Aufzeichnungsverfahren sowie für den Einsatz als ladungsverbesserndes Mittel in Pulvern und Lacken zur Oberflächenbeschichtung, insbesondere in triboelektrisch bzw. elektrokinetisch versprühten Pulverlacken.

In den folgenden Beispielen sind alle Schmelzpunktangaben unkorrigiert.

### Beispiel 1

30,6 g 2,2'-Dithiodibenzoesäure (0,10 Mol) werden in 600 ml Ethanol angeschlämmt. Zu dieser Anschlämmung gibt man unter Rühren bei 70 - 75°C langsam tropfenweise 36,5 g einer 25%igen wäßrigen Tetramethylammoniumhydroxidlösung (0,10 Mol). Aus der Reaktionslösung scheidet sich beim Einengen eine feuchte Kristallmasse ab, die im Umluftschrank bei 120°C getrocknet und anschließend gemahlen wird.

Ausbeute: 37,8 g (99,6% der Theorie) 2,2'-Dithiodibenzoesäure-mono(tetramethylammonium)salz der Formel 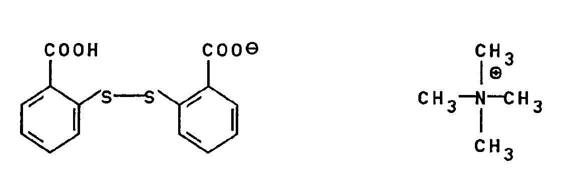
C₁₈H₂1NO₄S₂ Molekulargewicht: 379,49
Weißes Pulver
Schmelzpunkt: 240°C
1H-NMR (in DMSO-d₆): δ3,15 (s, 12H), 7,1-8,1 (m,8Hₐᵣ.)

### Beispiel 2

Es wird wie in Beispiel 1 verfahren, mit dem Unterschied, daß 72,9 g einer 25%igen wäßrigen Tetramethylammoniumhydroxidlösung (0,20 Mol) zugegeben werden.
Ausbeute: 45,2 g (99,9 % der Theorie)
2,2'Dithiodibenzoesäuredi(tetramethylammonium)salz der Formel 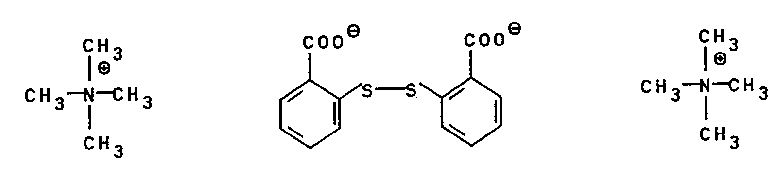
C₂₂H₃₂N₂O₄S₂ Molekulargewicht: 452,63
Weißes Pulver
Schmelzpunkt: 252°C
1H-NMR (in DMSO-d₆): δ3,17 (s, 24H), 6,9-7,9 (m,8H_{ar.})

### Beispiel 3

Es wird wie in Beispiel 1 verfahren, mit dem Unterschied, daß anstelle von Tetramethylammoniumhydroxidlösung 36,8 g einer 40%igen wäßrigen Tetraethylammoniumhydroxidlösung (0,10 Mol) verwendet werden.
Ausbeute: 43,3 g (99,4 % der Theorie) 2,2'-Dithiodibenzoesäuremono-(tetraethylammonium)salz der Formel 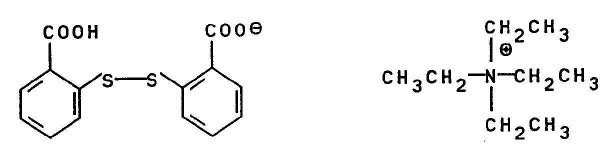
C₂₂H₂₉NO₄S₂ Molekulargewicht 435,60
Weißes Pulver
Schmelzpunkt: 255°C
1H-NMR (in DMSO-d₆): δ1,15 (t, 12H), 3,20 (g, 8H), 7,1-8,1 (m, 8H_{ar.})

### Beispiel 4

Es wird wie in Beispiel 1 verfahren, mit dem Unterschied, daß anstelle von Tetramethylammoniumhydroxidlösung 73,6 g einer 40%igen wäßrigen Tetraethylammoniumhydroxidlösung (0,20 Mol) verwendet werden.
Ausbeute: 56,3 g (99,7% der Theorie) 2,2'-Dithiodibenzoesäuredi(tetraethylammonium)salz der Formel 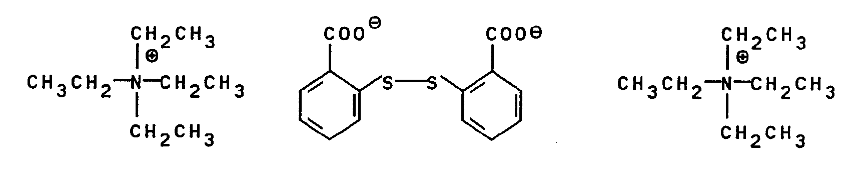
C₃₀H₄₈N₂O₄S₂ Molekulargewicht 564,84
Weißes Pulver
Schmelzpunkt: 160°C
1H-NMR (in DMSO-d₆): δ1,10 (t, 24H), 3,15 (q, 16H), 6,8-7,9 (m, 8H_{ar.})

### Beispiel 5

Es wird wie in Beispiel 1 verfahren, mit dem Unterschied, daß anstelle von Tetramethylammoniumhydroxidlösung 101,7 g einer 20 %igen wäßrigen Tetrapropylammoniumhydroxidlösung (0,10 Mol) verwendet werden.
Ausbeute: 48,7 g (99,0 % der Theorie) 2,2'-Dithiodibenzoesäuremono(tetrapropylammonium)salz der Formel 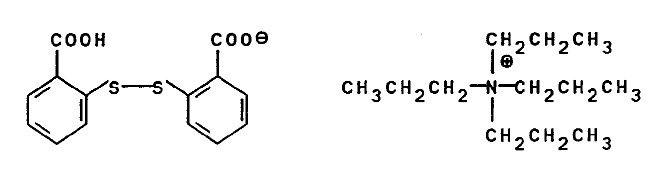
C₂₆H₃₇NO₄S₂ Molekulargewicht 491,70
Weißes Pulver
Schmelzpunkt: 255°C
1H-NMR (in DMSO-d₆): δ0,90 (t, 12H), 1,60 (m, 8H), 3,10 (m, 8H), 7,1-8,1 (m, 8H_{ar.})

### Beispiel 6

Es wird wie in Beispiel 1 verfahren, mit dem Unterschied, daß anstelle von Tetramethylammoniumhydroxidlösung 65 g einer 40%igen wäßrigen Tetrabutylammoniumhydroxidlösung (0,10 Mol) verwendet werden.
Ausbeute: 54,3 g (99,1 % der Theorie) 2,2'-Dithiodibenzoesäuremono(tetrabutylammonium)salz der Formel 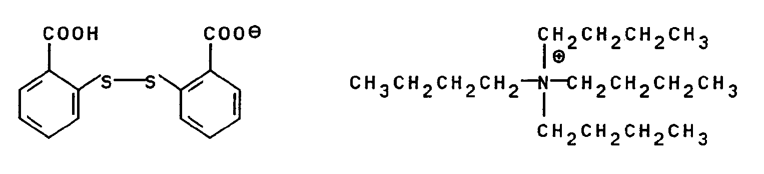
C₃₀H₄₅NO₄S₂ Molekulargewicht 547,81
Weißes Pulver
Schmelzpunkt: 228°C
1H-NMR (in DMSO-d₆): δ0,91 (t, 12H), 1,30 (m, 8H), 1,58 (m, 8H), 3,18 (m, 8H), 7,1-8,1 (m, 8H_{ar.})

### Beispiel 7

Es wird wie in Beispiel 1 verfahren, mit dem Unterschied, daß anstelle von Tetramethylammoniumhydroxidlösung 130 g einer 40%igen wäßrigen Tetrabutylammoniumhydroxidlösung (0,20 Mol) verwendet werden.
Ausbeute: 87,5 g (99,8 % der Theorie) 90 %iges
2,2'-Dithiodibenzoesäuredi(tetrabutylammonium)salz der Formel 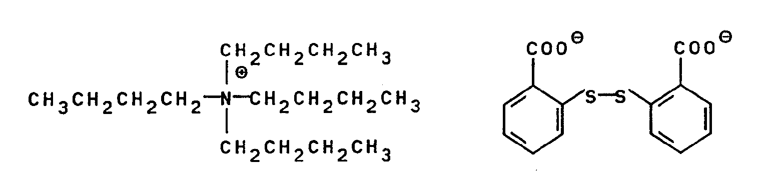 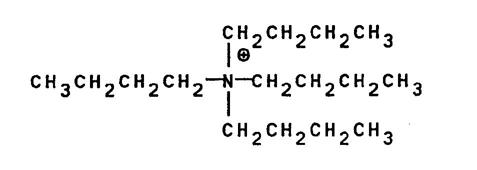
C₄₆H₈₀N₂O₄S₂ Molekulargewicht 789,27
Zähfließendes Öl, nach längerem Stehen an der Luft wachsartige Masse mit 10 Massen % Wasser
Schmelzpunkt: ca. 45°C
1H-NMR (in DMSO-d₆): δ0,92 (t, 24H); 1,30 (m, 16H), 1,58 (m, 16H), 3,19 (m, 16H), 6,95-7,85 (m, 8H_{ar.})

### Beispiel 8

In eine Lösung von 8,00 g Natriumhydroxid (0,20 Mol) in 800 ml Wasser werden bei Raumtemperatur unter Rühren 30,6 g 2,2'-Dithiodibenzoesäure (0,10 Mol) eingetragen und gelöst. Zu dieser Lösung gibt man eine Lösung von 26,6 g Tetrapropylammoniumbromid (0,10 Mol) in 300 ml Wasser. Anschließend wird eine Lösung von 3,65 g Chlorwasserstoff (0,10 Mol) in 100 ml Wasser langsam zugetropft, wobei das Produkt ausfällt. Die Reaktionsmischung wird 15 Stunden nachgerührt. Dann wird der Niederschlag abgesaugt, mit Wasser gewaschen und im Umluftschrank bei 120°C getrocknet.
Ausbeute: 48,3 g (98,2% der Theorie) 2,2'-Dithiodibenzoesäuremono(tetrapropylammonium)salz der im Beispiel 5 wiedergegebenen Formel.
Weißes Pulver
Schmelzpunkt: 258°C
1H-NMR (in DMSO-d₆): übereinstimmend mit dem Spektrum des Produkts des Beispiels 5.

### Beispiel 9

Es wird wie in Beispiel 8 verfahren, mit dem Unterschied, daß anstelle von Tetrapropylammoniumbromid 32,2 g Tetrabutylammoniumbromid (0,10 Mol) verwendet werden.
Ausbeute: 53,9 g (98,4 % der Theorie) 2,2'-Dithiodibenzoesäuremono(tetrabutylammonium)salz der im Beispiel 6 wiedergegebenen Formel.
Weißes Pulver
Schmelzpunkt: 230°C
1H-NMR (in DMSO-d₆): übereinstimmend mit dem Spektrum des Produkts des Beispiels 6.

### Beispiel 10

Es wird wie in Beispiel 8 verfahren, mit dem Unterschied, daß anstelle von Tetrapropylammoniumbromid 33,9 g Tetrabutylphosphoniumbromid (0,10 Mol) verwendet werden.
Ausbeute: 55,3 g (97,9 % der Theorie) 2,2'-Dithiodibenzoesäuremono(tetrabutylphosphonium)salz der Formel 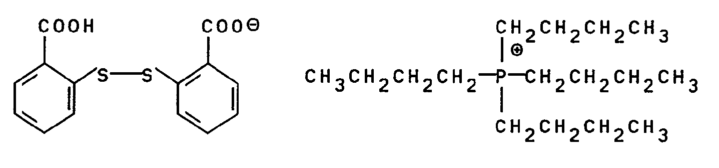
C₃₀H₄5O₄PS₂ Molekulargewicht 564,78
Weißes Pulver
Schmelzpunkt: 243°C
1H-NMR (in DMSO-d₆): δ0,95 (t, 12H), 1,44 (m, 16H), 2,20 (m, 8H), 7,15-8,05 (m, 8H_{ar.})

### Beispiel 11

Es wird wie in Beispiel 1 verfahren, mit dem Unterschied, daß anstelle von Tetramethylammoniumhydroxidlösung 54,4 g einer 40%igen wäßrigen Tributylmethylammoniumhydroxidlösung (0,10 Mol) verwendet werden.
Ausbeute: 50,3 g (99,5 % der Theorie) 2,2'Dithiodibenzoesäuremono(tributylmethylammonium)salz der Formel 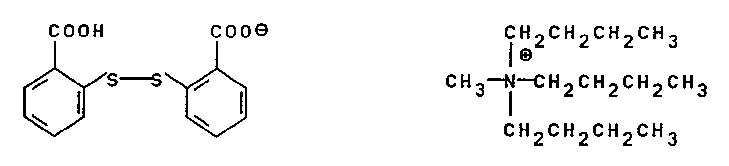
C₂₇H₃₉NO₄S₂ Molekulargewicht 505,73
Weißes Pulver
Schmelzpunkt: 198°C
1H-NMR (in DMSO-d₆): δ0,90 (t, 9H), 1,28 (m, 6H), 1,59 (m, 6H); 2,96 (s, 3H), 3,20 (m, 6H); 7,1-8,1 (m, 8H_{ar.})

### Beispiel 12

Es wird wie in Beispiel 1 verfahren, mit dem Unterschied, daß anstelle von Tetramethylammoniumhydroxidlösung 47,7 g einer 35%igen methanolischen Benzyltrimethylammoniumhydroxidlösung (0,10 Mol) verwendet werden.
Ausbeute: 45,4 g (99,6 % der Theorie) 2,2'-Dithiodibenzoesäuremono(benzyltrimethylammonium)salz der Formel 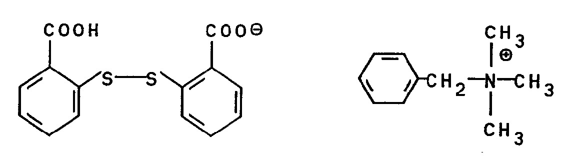
C₂₄H₂5NO₄S₂ Molekulargewicht 455,59
Weißes Pulver
Schmelzpunkt: 164°C
1H-NMR (in DMSO-d₆): δ3,07 (s, 9H), 4,59 (s, 2H), 7,1-8,1 (m, 13 H_{ar.})

### Beispiel 13

47 g Natriumhydroxid werden in 750 ml Wasser gelöst. In die abgekühlte Natronlauge werden 180 g technische 2,2'-Dithiodibenzoesäure mit einem Reingehalt von 85 Gew.% eingerührt und gelöst. Die Lösung wird von Schmutzpartikeln filtriert. Zu dem Filtrat werden 296 g 45%ige wäßrige Tetrapropylammoniumbromidlösung gegeben. Anschließend wird unter Rühren gasförmiges Kohlendioxid eingeleitet, bis der pH-Wert 7,5 ist. Man rührt eine Stunde lang nach und saugt anschließend den Feststoff über eine Filternutsche ab. Das Produkt wird mit 3000 ml Wasser gewaschen, gut trockengesaugt und 24 Stunden bei 100°C im Vakuum getrocknet.
Ausbeute: 222 g (90% der Theorie), 2,2'-Dithiodibenzoesäuremono(tetrapropylammonium)salz.
C₂₆H₃₇NO₄S₂ Molekulargewicht 491,70
Schmelzpunkt: 250°C
1H-NMR (in DMSO-d6): δ0,9 (t, 12H), 1,6 (m, 8H), 3,1 (m, 8H), 7,1-8,1 (m, 8 Hₐᵣ).

### Beispiel 14

Es wird wie in Beispiel 13 verfahren mit dem Unterschied, daß anstelle der Tetrapropylammoniumbromidlösung 377 g 45%ige wäßrige Tetrabutylphosphoniumbromidlösung zugegeben werden und Kohlendioxid eingeleitet wird, bis der pH-Wert 7,0 ist.
Ausbeute: 277,5 g (98,4 % der Theorie)
2,2'-Dithiodi-benzoesäuremono(tetrabutylphosphonium)salz.
C₃₀H₄₅O₄PS₂ Molekulargewicht 564,78
Schmelzpunkt: 237°C
¹H-NMR (in DMSO-d₆): δ0,95 (t, 12H), 1,45 (m, 16H), 2,2 (m, 8H), 7,1-8,1 (m, 8Hₐᵣ).

## Patentansprüche

1. Verbindungen der allgemeinen Formel I 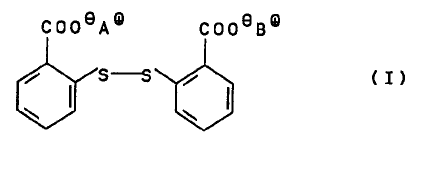 worin
A^{⊕} und B^{⊕} unabhängig voneinander mit der Einschränkung, daß sie nicht beide gleichzeitig Protonen bedeuten, ein Proton oder ein Ion der allgemeinen Formel II 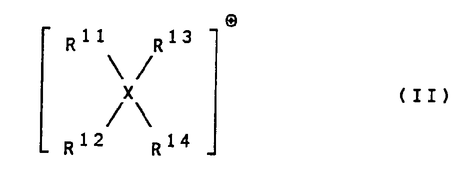 worin X für Stickstoff oder Phosphor steht; und R¹¹, R¹², R¹³ und R¹⁴ unabhängig voneinander mit der Einschränkung, daß sie nicht alle gleichzeitig für Wasserstoff stehen, für Wasserstoff; (C₁-C₃₀)-Alkyl; (C₅-C₁₂)-Cycloalkyl; (C₆-C₁₂)-Aryl oder für (C₆-C₁₂)-Aryl-(C₁-C₆)-alkyl stehen.

2. Verbindung gemäß Anspruch 1, dadurch gekennzeichnet, daß sie die allgemeine Formel Ib 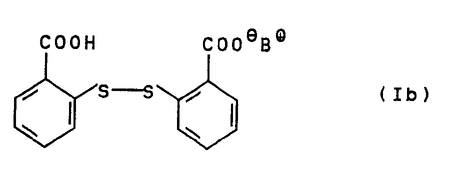 besitzt,
worin B^{⊕}
Tetramethylammonium
Tetraethylammonium
Tetrapropylammonium
Tetrabutylammonium
Tetrapentylammonium
Tetrahexylammonium
Tetraheptylammonium
Tetraoctylammonium
Tetranonylammonium
Tetrakis-(decyl)-ammonium
Tetradodecylammonium
Tributylmethylammonium
Benzyltrimethylammonium
Ethylhexadecyldimethylammonium
Benzyldimethylhexadecylammonium
Benzyltriethylammonium
Hexadecyltrimethylammonium
Tetrapropylphosphonium
Tetrabutylphosphonium
Tetrapentylphosphonium
Benzyltriphenylphosphonium oder
Hexadecyltributylphosphonium
bedeutet.

3. Verbindung gemäß Anspruch 1 und/oder 2, dadurch gekennzeichnet, daß sie die allgemeine Formel Ic 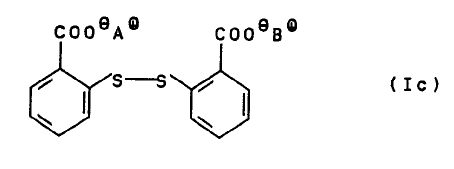 besitzt,
worin A^{⊕} und B^{⊕} identisch sind und wie B^{⊕} in Anspruch 2 definiert sind.

4. Gemische sowie Mischkristalle aus
a) 10 bis 100 Gew.% einer oder mehreren Verbindungen der allgemeinen Formel I gemäß Anspruch 1 und
b) 0 bis 90 Gew.% 2,2'-Dithiodibenzoesäure.

5. Verfahren zur Herstellung einer Verbindung der allgemeinen Formel I gemäß Anspruch 1 durch Umsetzung einer Verbindung der allgemeinen Formel V 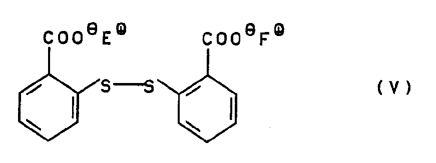 worin
E^{⊕} und F^{⊕} unabhängig voneinander für ein Proton, ein Alkaliion oder das Ammoniumion stehen, mit einer Verbindung der Formel A^{⊕}Yₐ^{⊖} oder B^{⊕}Y_{b}^{⊖} oder einem Gemisch beider Verbindungen,
worin Yₐ^{⊖} und Y_{b}^{⊖} unabhängig voneinander ein Anion oder ein Anionäquivalent bedeuten und A^{⊕} und B^{⊕} die in Anspruch 1 angegebene Bedeutung hat, wobei das Proton ausgeschlossen ist.

6. Verfahren zur Herstellung von in Wasser schwerlöslichen Monosalzen der allgemeinen Formel I gemäß Anspruch 1, dadurch gekennzeichnet, daß sie aus einer wäßrigen lösung, die eine Verbindung der allgemeinen Formel Va 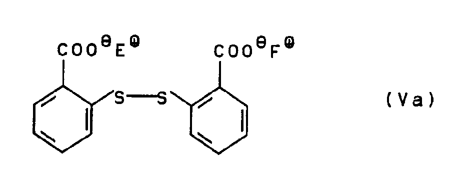 worin
E^{⊕} und F^{⊕} unabhängig voneinander für ein Alkaliion oder das Ammoniumion stehen und eine Verbindung A^{⊕}Yₐ^{⊖} oder B^{⊕}Y_{b}^{⊖}, worin A^{⊕} und B^{⊕} wie in Anspruch 1 angegeben definiert sind und Yₐ^{⊖} und Y_{b}^{⊖} ein Anion oder ein Anionäquivalent bedeuten, in äquimolaren Mengen enthält, durch Kohlendioxid gefällt werden.

7. Verwendung der Verbindungen der allgemeinen Formel I des Anspruchs 1 als Ladungssteuermittel in Tonern und Entwicklern für elektrophotographische Aufzeichnungsverfahren sowie als ladungsverbesserndes Mittel in Pulvern und Lacken zur Oberflächenbeschichtung.

## Claims

1. Compounds of the general formula I 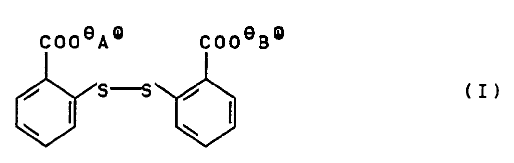 wherein A^{⊕} and B^{⊕} independently of one another denote a proton, with the limitation that they do not both simultaneously denote protons, or an ion of the general formula II 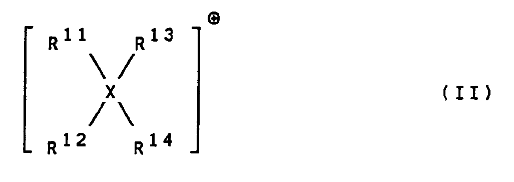 wherein X represents nitrogen or phosporus; and R¹¹, R¹², R¹³ and R¹⁴ independently of one another represent hydrogen, with the limitation that they do not all simultaneously represent hydrogen; (C₁-C₃)-alkyl; (C₅-C₁₂)-cycloalkyl; (C₆-C₁₂)-aryl or (C₆-C₁₂)-aryl-(C₁-C₆)-alkyl.

2. Compound according to Claim 1, characterised in that it has the general formula Ib 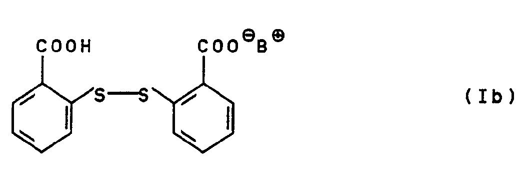 wherein B^{⊕} denotes
tetramethylammonium,
tetraethylammonium,
tetrapropylammonium,
tetrabutylammonium,
tetrapentylammonium,
tetrahexylammonium,
tetraheptylammonium,
tetraoctylammonium,
tetranonylammonium,
tetrakis-(decyl)-ammonium,
tetradodecylammonium,
tributylmethylammonium,
benzyltrimethylammonium,
ethylhexadecyldimethylammonium,
benzyldimethylhexadecylammonium,
benzyltriethylammonium,
hexydecyltrimethylammonium,
tetrapropylphosphonium,
tetrabutylphosphonium,
tetrapentylphosphonium,
benzyltriphenylphosphonium or
hexadecyltributylphosphonium.

3. Compound according to Claim 1 and/or 2, characterised in that it has the general formula Ic 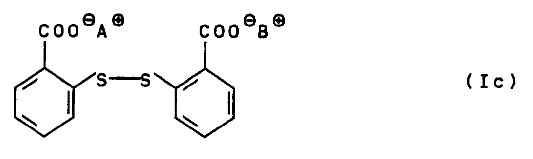 wherein A^{⊕} and B^{⊕} are identical and B^{⊕} is
defined as in Claim 2.

4. Mixtures and mixed crystals of
a) 10 to 100 % by weight of one or more compounds of the general formula I of Claim 1 and
b) 0 to 90 % by weight of 2,2'-dithiodibenzoic acid.

5. Process for the preparation of a compound of the general formula I of Claim 1, by reaction of a compound of the general formula V 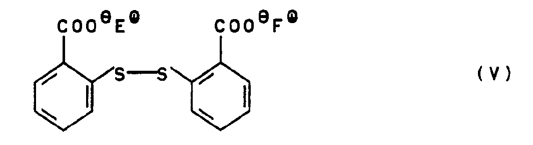 wherein E^{⊕} and F^{⊕} independently of one another represent a proton, an alkali metal ion or the ammonium ion, with a compound of the formula A^{⊕}Yₐ^{⊖} or B^{⊕}Y_{b}^{⊖}, or a mixture of the two compounds, wherein Yₐ^{⊖} and Y_{b}^{⊖} independently of one another denote an anion or an anion equivalent and A^{⊕} and B^{⊕} have the meaning given in Claim 1, the proton being excluded.

6. Process for the preparation of sparingly water-soluble mono-salts of the general formula I of Claim 1, characterised in that they are precipitated, by means of carbon dioxide, from an aqueous solution which contains equimolar amounts of a compound of the general formula Va 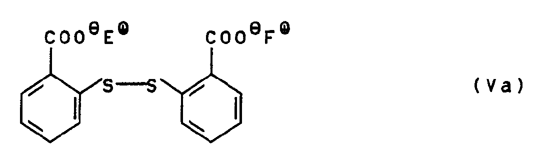 wherein E^{⊕} and F^{⊕} independently of one another represent an alkali metal ion or the ammonium ion, and a compound A^{⊕}Yₐ^{⊖} or B^{⊕}Y_{b}^{⊖}, wherein A^{⊕}
and B^{⊕} are defined as in Claim 1 and Yₐ^{⊖} and
Y_{b}^{⊖} denote an anion or an anion equivalent.

7. Use of the compounds of the general formula I of Claim 1 as charge control agents in toners and developers for electrophotographic recording processes and as charge improving agents in powders and varnishes for surface coating.

## Revendications

1. Composés de formule générale I 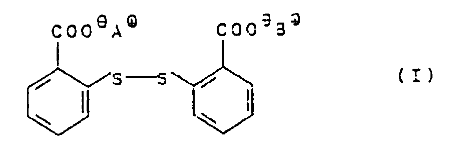 dans laquelle
A^{⊕} et B^{⊕}, indépendamment l'un de l'autre, et à condition qu'ils ne représentent pas tous les deux et simultanément des protons, représentent chacun un proton ou un ion de formule générale II 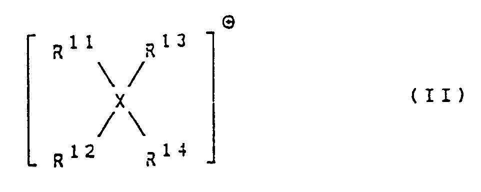 dans laquelle X est un atome d'azote ou de phosphore ; et R¹¹, R¹², R¹³ et R¹⁴, indépendamment les uns des autres, et à condition qu'ils ne soient pas tous simultanément des atomes d'hydrogène, représentent chacun un hydrogène ou un radical alkyle en C₁-C₃₀, cycloalkyle en C₅-C₁₂, aryle en C₆-C₁₂ ou (aryle en C₆-C₁₂)-(alkyle en C₁-C₆).

2. Composé selon la revendication 1, caractérisé en ce qu'il répond à la formule générale Ib 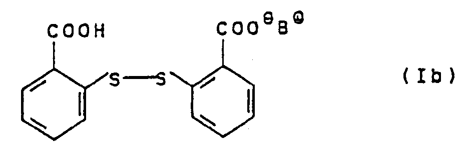 dans laquelle B^{⊕} est l'un des ions suivants :
tétraméthylammonium
tétraéthylammonium
tétrapropylammonium
tétrabutylammonium
tétrapentylammonium
tétrahexylammonium
tétraheptylammonium
tétraoctylammonium
tétranonylammonium
tétrakys-(décyl)-ammonium
tétradodécylammonium
tributylméthylammonium
benzyltriméthylammonium
éthylhexadécyldiméthylammonium
benzyldiméthylhexadécylammonium
benzyltriéthylammonium
hexadécyltriméthylammonium
tétrapropylphosphonium
tétrabutylphosphonium
tétrapentylphosphoniumm
benzyltriphénylphosphonium ou
hexadécyltributylphosphonium.

3. Composé selon les revendications et/ou 2, caractérisé en ce qu'il répond à la formule générale Ic 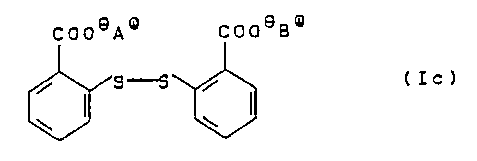 dans laquelle A^{⊕} et B^{⊕} sont identiques et sont tels que définis pour B^{⊕} dans la revendication 2.

4. Mélanges et solutions solides constitués
a) de 10 à 100 % en poids d'un ou plusieurs composés de formule générale I selon la revendication 1, et
b) de 0 à 90 % en poids d'acide 2,2'-dithiodibenzoïque.

5. Procédé pour préparer un composé de formule générale I selon la revendication 1, par réaction d'un composé de formule générale V 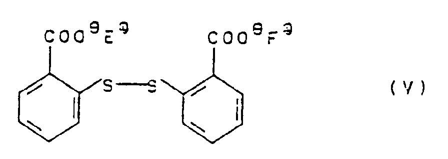 dans laquelle E^{⊕} et F^{⊕}, indépendamment l'un de l'autre, sont chacun un proton, un ion de métal alcalin ou l'ion ammonium, avec un composé de formule A^{⊕}Yₐ^{⊖} ou B^{⊕}Y_{b}^{⊖} ou encore avec un mélange de ces deux composés, où Yₐ^{⊖} et Y_{b}^{⊖} étant, indépendamment l'un de l'autre, chacun un anion ou un équivalent d'anion, et A^{⊕} et B^{⊕} ont les significations données dans le revendication 1 à l'exclusion du proton.

6. Procédé pour préparer des monosels difficilement solubles dans l'eau, de formule générale I selon la revendication 1, caractérisé en ce qu'on les précipite à l'aide d'anhydride carbonique, à partir d'une solution aqueuse contenant en des quantités équimolaires un composé de formule générale Va 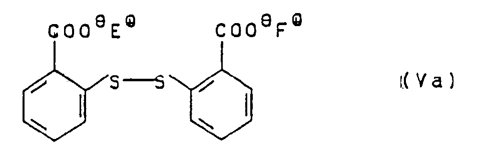 dans laquelle
E^{⊕} et F^{⊕}, indépendamment l'un de l'autre, sont chacun un ion de métal alcalin ou l'ion ammonium, et un composé A^{⊕}Yₐ^{⊖} ou B^{⊕}Y_{b}^{⊖} où A^{⊕} et B^{⊕} sont tels que définis dans la revendication 1 et Yₐ^{⊖} et Y_{b}^{⊖} représentent chacun un anion ou un équivalent d'anion.

7. Utilisation des composés de formule générale I selon la revendication 1, en tant que régulateurs de charge dans des toners et des révélateurs pour des procédés d'enregistrement électro-photographique et également en tant que moyens améliorant la charge dans les poudres et vernis utilisés pour le revêtement des surfaces.
